(19) Europäisches Patentamt
European Patent Office
Office européen des brevets

(11) Veröffentlichungsnummer: **0 385 369 B1**

(12) **EUROPÄISCHE PATENTSCHRIFT**

(45) Veröffentlichungstag der Patentschrift: **20.09.95**

(21) Anmeldenummer: **90103737.4**

(22) Anmeldetag: **26.02.90**

(51) Int. Cl.6: **C11D 1/40,** C11D 3/00,
C11D 3/30, A61K 7/48,
A61K 7/08, A01N 33/04,
C10M 173/02

(54) **Verfahren zur antimikrobiellen Konservierung von Tensiden.**

(30) Priorität: **27.02.89 CH 718/89**

(43) Veröffentlichungstag der Anmeldung:
**05.09.90 Patentblatt 90/36**

(45) Bekanntmachung des Hinweises auf die
Patenterteilung:
**20.09.95 Patentblatt 95/38**

(84) Benannte Vertragsstaaten:
**AT BE CH DE ES FR GB IT LI NL**

(56) Entgegenhaltungen:
**EP-A- 0 333 143**
**EP-A- 0 343 605**

(73) Patentinhaber: **LONZA AG**
**(Geschäftsleitung: 4002 Basel)**
**CH-3945 Gampel/Wallis (CH)**

(72) Erfinder: **Güller, Siegfried, Dr., Ing-Chem.**
**Starenstrasse 1**
**Bottmingen (Kanton Baselland) (CH)**
Erfinder: **Fritschi, Joachim, Dipl.-Ing.**
**Im Altweg 13B**
**D-7850 Lörrach (DE)**
Erfinder: **Lichtenberg, Florian, Dr., Dipl.-Ing.**
**Röttler Ring 12**
**D-7889 Grenzach (DE)**

(74) Vertreter: **Weinhold, Peter, Dr. et al**
**Patentanwälte**
**Dr. Weinhold, Dannenberg,**
**Dr. Gudel, Schubert**
**Siegfriedstrasse 8**
**D-80803 München (DE)**

## Beschreibung

Die vorliegende Erfindung betrifft ein Verfahren zur antimikrobiellen Konservierung von flüssigen Tensiden sowie tensidhaltigen Lösungen und anderen tensidhaltigen Zubereitungen, die bei der Lagerung oder dem Gebrauch durch Mikrobenbefall beeinträchtigt werden können.

Der Schutz von flüssigen Tensiden, worunter im wesentlichen konzentrierte wässrige Lösungen zu verstehen sind, sowie allgemein von tensidhaltigen Lösungen oder Zubereitungen vor Befall durch Bakterien, Hefen und Schimmelpilze und dem dadurch bedingten Verderb ist ein bedeutendes technisches Problem. Insbesondere die allgemein geforderte gute biologische Abbaubarkeit solcher Tenside in verdünnter wässriger Lösung bedingt naturgemäss auch einen leichten und raschen mikrobiellen Befall und Verderb, sobald Wasser anwesend ist. Das betrifft vor allem solche Lösungen oder Zubereitungen, die bei ihrem Gebrauch Umwelteinflüssen direkt ausgesetzt sind und dabei zwangsläufig mit verschiedenartigen Mikroorganismen kontaminiert werden. Dazu gehören beispielsweise Kühlschmierstoffe, die auch unter der Bezeichnung Bohr- oder Schneidöl bei der spanabhebenden Metallbearbeitung verwendet werden.

Solche Kühlschmierstoffe bestehen zu einem wesentlichen Teil aus anionischen Tensiden wie Seifen unterschiedlichster Art, nichtionogenen Tensiden wie höhere ethoxylierte Fettalkohole, sowie anderen organischen Verbindungen, die wenigstens teilweise biologisch abbaubar sind. Es handelt sich dabei ebenfalls um wässrige Systeme, die deshalb sehr leicht von Mikroorganismen befallen und zersetzt werden. Hierbei können sich schleimige Ablagerungen bilden, welche die Funktion der Kühl- und Umwälzvorrichtungen, wie Düsen, Leitungen, Filter und Pumpen, beeinträchtigen. Die Zersetzungsprodukte und die Ausscheidungen der Mikroorganismen setzen die Wirksamkeit der Kühlschmiermittel herab und führen zu Geruchsbelästigungen oder sogar gesundheitlichen Gefährdungen des Personals, das zwangsläufig mit den Kühlschmiermitteln in Kontakt kommt. Daher ist es unbedingt erforderlich, diese Mittel in der gebrauchsfertigen Verdünnung zu konservieren.

Aehnliche Probleme treten beim Gebrauch von Kettengleitmitteln auf, die ebenfalls überwiegend aus Tensiden bestehen und vor allem in der Nahrungsmittel- bzw. Getränkeindustrie zum Schmieren von Transportvorrichtungen, beispielsweise an Abfüllmaschinen, eingesetzt werden.

Zur Erzielung einer ausreichenden Haltbarkeit dieser Produkte ist daher im allgemeinen der Zusatz von Konservierungsmitteln unumgänglich.

Zu diesem Zweck wurde früher in erster Linie Formaldehyd verwendet, und zwar sowohl in Substanz als auch in Form von Derivaten, die diesen allmählich wieder freisetzen (Formaldehydabspalter). Aufgrund seiner toxikologischen Eigenschaften und der Sensibilisierung weiter Konsumentenkreise wird jedoch die Verwendung von Formaldehyd zumindest in Produkten für den Haushaltgebrauch nicht mehr akzeptiert.

Andere geeignete Konservierungsmittel sind zwar bekannt, jedoch häufig nur unter bestimmten Bedingungen ausreichend wirksam oder für den Einsatz in grösserem Umfang zu teuer.

Aufgabe der Erfindung war daher, ein Verfahren zur antimikrobiellen Konservierung von flüssigen Tensiden oder tensidhaltigen Lösungen oder Zubereitungen zur Verfügung zu stellen, das ohne Verwendung von Formaldehyd oder anderen Aldehyden auskommt und auf kostengünstige Weise einen breiten Anwendungsbereich abdeckt.

Erfindungsgemäss wird diese Aufgabe durch ein Verfahren gemäss Patentanspruch 1 gelöst.

Das N,N-Bis-(3-aminopropyl)-laurylamin wird den zu konservierenden Tensiden oder tensidhaltigen Lösungen oder Zubereitungen zweckmässig in einer Menge von 0,01 bis 1,0 Gew.%, vorzugsweise 0,02 bis 0,5 Gew.%, bezogen auf die fertige Mischung, zugesetzt.

Die Zugabe kann sowohl in reiner Form als auch vorzugsweise in wässriger Lösung erfolgen. Besonders bevorzugt ist eine 20-40%ige wässrige Lösung.

Es ist auch möglich, Gemische mit anderen N,N-Bis-(3-aminopropyl)-alkylaminen zu verwenden, wenn diese ausreichende Mengen N,N-Bis-(3-aminopropyl)-laurylamin enthalten. Solche Gemische können beispielsweise aus entsprechenden technischen Fettamingemischen, wie insbesondere Kokosamin, nach bekannten Verfahren hergestellt werden.

Es liegt weiterhin im Rahmen der Erfindung, das N,N-Bis-(3-aminopropyl)-laurylamin als Bestandteil eines antimikrobiellen Konservierungsmittels, das gegebenenfalls noch weitere antimikrobiell wirksame Substanzen und/oder sonstige Hilfsstoffe, wie Farbstoffe, Duftstoffe, Lösungsmittel und/oder weitere Zusätze enthält, bei der Durchführung des erfindungsgemässen Verfahrens einzusetzen.

Das erfindungsgemässe Verfahren eignet sich vorzugsweise zur antimikrobiellen Konservierung von anionischen flüssigen Tensiden, wie Seifen, also beispielsweise Natriumstearat, Kaliumstearat oder Triethanolaminseifen, sulfonierte aromatische Kohlenwasserstoffe, wie n-Alkylbenzolsulfonate, sulfonierte aliphatische Kohlenwasserstoffe, wie sekundäre Alkansulfonate, sulfonierte $\alpha$-Olefine, sulfatierte Fettalkohole, wie Natriumlaurylsulfat, sulfatierte Fettalkoholether, wie Natriumlaurylpolyglykolethersulfat, sulfonierte Fettsäuremethylesterpolyglykolether, wie Laurylpolyglykoletheracetat, und zwar sowohl einzeln als auch in Kombination, deren Lösungen und sonstigen Zubereitungen wie beispielsweise Haushaltsreiniger, flüssige Wasch- und Geschirrspülmittel, Weichspüler und Autoshampoos.

Eine besonders bevorzugte Anwendung des Verfahrens ist die Konservierung von flüssigen tensidhaltigen Körperpflegemitteln wie Körperreinigungsmittel, Bade- und Duschzusätze, Haarshampoos und Körperlotionen.

Insbesondere eignet sich das Verfahren auch zur Konservierung von Kühlschmierstoffen, die auch unter der Bezeichnung Bohr- oder Schneidöl bei der spanabhebenden Metallverarbeitung verwendet werden.

Eine weitere besonders bevorzugte Anwendung des erfindungsgemässen Verfahrens ist die Konservierung von Kettengleitmitteln.

Die nachfolgenden Beispiele verdeutlichen die Ausführung der Erfindung und die Wirksamkeit des erfindungsgemässen Verfahrens.

Beispiel 1

Eine 30%ige wässrige Lösung der Magnesiumsalze eines $C_{12/14}$-Fettalkoholsulfatgemisches wurde mit 0,05 bis 0,5% einer 30%igen wässrigen Lösung von N,N-Bis-(3-aminopropyl)-laurylamin (entsprechend 0,015 bis 0,15% Wirkstoff) versetzt und den Konservierungsbelastungstests nach DAB 9 unterzogen. Als Testkeime dienten E.coli ATCC 8739, Pseudomonas aeruginosa ATCC 9027, Straphylococcus aureus ATCC 6538, Candida albicans ATCC 10231 und Aspergillus niger ATCC 16404. Zum Vergleich wurde jeweils eine Probe ohne Wirkstoffzusatz getestet.

Die Ergebnisse der quantitativen und qualitativen Reisolierung nach 24 Stunden bis 21 Tagen sind in Tabelle 1 zusammengestellt.

S.aureus und C.albicans wurden auch ohne Wirkstoffzusatz innerhalb von 24 Stunden quantitativ eliminiert und sind deshalb nicht in die Tabelle aufgenommen.

Beispiel 2

Mit einer 28%igen wässrigen Lösung eines Natriumlaurylether sulfats mit 3 Mol Ethylenoxid wurden die gleichen Tests wie in Beispiel 1 durchgeführt.

Die Ergebnisse sind in Tabelle 2 aufgeführt.

S.aureus wurde nicht in die Tabelle aufgenommen, da er bereits ohne Wirkstoffzusatz innerhalb von 24 Stunden vollständig eliminiert wurde.

Beispiel 3

Mit einer 4%igen wässrigen Lösung von Distearyldimethylammoniumchlorid, die in der Zusammensetzung einem der handelsüblichen Weichspülerkonzentrate entspricht, wurden die gleichen Tests wie in Beispiel 1 durchgeführt.

Die Ergebnisse sind der Tabelle 3 zu entnehmen.

Auch hier wurde S.aureus ohne Wirkstoffzusatz innerhalb von 24 Stunden vollständig eliminiert und daher nicht in die Tabelle aufgenommen.

Beispiel 4

Es wurde ein Badeshampoo gemäss folgender Rezeptur hergestellt (Angaben in Gew.%):
20,0% Monoisopropanolammonium-Fettalkoholsulfat
0,5% Duftstoff
0,3% N,N-Bis-(3-aminopropyl)-laurylamin
ad 100% Wasser
Die fertige Formulierung wurde wie in Beispiel 1 dem Konservierungsbelastungstest nach DAB 9 unterzogen. Die Ergebnisse sind in Tabelle 4 zusammengefasst.

Beispiel 5

Es wurde ein Schaumbad gemäss folgender Rezeptur hergestellt (Angaben in Gew.%):
8,0% Ammoniumalkylethersulfat
3,0% 1,2-Propylenglykol

EP 0 385 369 B1

2,0% Kokosfettsäurediethanolamid
0,5% Duftstoff
0,05% Zitronensäure
0,3% N,N-Bis-(3-aminopropyl)-laurylamin
ad 100% Wasser
Die fertige Formulierung wurde wie in Beispiel 1 dem Konservierungsbelastungstest nach DAB 9 unterzogen. Die Ergebnisse sind in Tabelle 5 zusammengefasst.

Beispiel 6

Es wurde eine Flüssigseife gemäss folgender Rezeptur hergestellt (Angaben in Gew.%):
13,0% Monoisopropanolammonium-Fettalkoholethersulfat
2,0% Kokosfettsäurediethanolamid
0,3% Duftstoff
0,3% N,N-Bis-(3-aminopropyl)-laurylamin
ad 100% Wasser
Die fertige Formulierung wurde wie in Beispiel 1 dem Konservierungsbelastungstest nach DAB 9 unterzogen. Die Ergebnisse sind in Tabelle 6 zusammengefasst.

4

# T a b e l l e 1

| Testkeime | Anfangsbelast. (KBE/ml) | Wirkstoff-zusatz (%) | Ergebnis der Reisolierung (Keimzahl/ml) nach: | | | |
|---|---|---|---|---|---|---|
| | | | 24 Std. | 7 Tagen | 14 Tagen | 21 Tagen |
| E.coli ATCC 8739 | 260000 | 0 | 300000/++ | 280000/++ | 9600/++ | 1,2 Mio/++ |
| | | 0,015 | 208000/++ | 56000/++ | 3600/++ | 17000/++ |
| | | 0,03 | 28000/++ | 4200/++ | 110/+ | 0/- |
| | | 0,075 | 270/++ | 0/- | 0/- | 0/- |
| | | 0,15 | 0/- | 0/- | 0/- | 0/- |
| Pseudomonas aerugi-nosa ATCC 9027 | 223000 | 0 | 180000/++ | 42000/++ | 1 Mio/++ | 2,7 Mio/++ |
| | | 0,015 | 11600/++ | 4500/++ | 100/++ | 17000/++ |
| | | 0,03 | 270/++ | 0/- | 0/- | 0/- |
| | | 0,075 | 0/- | 0/- | 0/- | 0/- |
| | | 0,15 | 0/- | 0/- | 0/- | 0/- |
| Aspergillus niger ATCC 16404 | 133000 | 0 | 21000/++ | 7200/++ | 110/++ | 0/- |
| | | 0,015 | 24000/++ | 780/++ | 0/- | 0/- |
| | | 0,03 | 40500/++ | 4000/++ | 100/+ | 0/- |
| | | 0,075 | 16200/++ | 990/++ | 100/+ | 0/- |
| | | 0,15 | 21000/++ | 1800/++ | 430/++ | 210/++ |

EP 0 385 369 B1

## T a b e l l e  2

EP 0 385 369 B1

| Testkeime | Anfangsbelast. (KBE/ml) | Wirkstoff-zusatz (%) | Ergebnis der Reisolierung (Keimzahl/ml)nach: | | | |
|---|---|---|---|---|---|---|
| | | | 24 Std. | 7 Tagen | 14 Tagen | 21 Tagen |
| E.coli ATCC 8739 | 260000 | 0 | 360000/++ | 790000/++ | 2,2 Mio/++ | 10 Mio/++ |
| | | 0,015 | 3200/++ | 103000/++ | 2,2 Mio/++ | 10 Mio/++ |
| | | 0,03 | 270/++ | 98000/++ | 2,4 Mio/++ | 7,4 Mio/++ |
| | | 0,075 | 0/– | 0/– | 0/– | 0/– |
| | | 0,15 | 0/– | 0/– | 0/– | 0/– |
| Pseudomonas aerugi-nosa ATCC 9027 | 223000 | 0 | 84000/++ | 190000/++ | 1 Mio/++ | 10 Mio/++ |
| | | 0,015 | 0/– | 0/– | 0/– | 0/– |
| | | 0,03 | 0/– | 0/– | 0/– | 0/– |
| | | 0,075 | 0/– | 0/–· | 0/– | 0/– |
| | | 0,15 | 0/– | 0/– | 0/– | 0/– |
| Candida albicans ATCC 10231 | 235000 | 0 | 68000/++ | 19500/++ | 7300/++ | 650/+ |
| | | 0,015 | 12000/++ | 7800/++ | 2800/++ | 1900/++ |
| | | 0,03 | 13000/++ | 460/++ | 0/– | 0/– |
| | | 0,075 | 0/– | 0/– | 0/– | 0/– |
| | | 0,15 | 0/– | 0/– | 0/– | 0/– |
| Aspergillus niger ATCC 16404 | 133000 | 0 | 214000/++ | 197000/++ | 160000/++ | 92000/++ |
| | | 0,015 | 240000/++ | 187000/++ | 150000/++ | 110000/++ |
| | | 0,03 | 180000/++ | 156000/++ | 130000/++ | 130000/++ |
| | | 0,075 | 38000/++ | 960/++ | 100/+ | 100/++ |
| | | 0,15 | 540/+ | 100/+ | 100/++ | 0/– |

## Tabelle 3

| Testkeime | Anfangsbelast. (KBE/ml) | Wirkstoff- zusatz (%) | Ergebnis der Reisolierung (Keimzahl/ml)nach: | | |
|---|---|---|---|---|---|
| | | | 24 Std. | 7 Tagen | 14 Tagen |
| E.coli ATCC 8739 | 208000 | 0 | 31000/++ | 60000/++ | 180000/++ |
| | | 0,015 | 0/- | 0/- | 0/- |
| | | 0,03 | 0/- | 0/- | 0/- |
| | | 0,15 | 0/- | 0/- | 0/- |
| Pseudomonas aerugi- nosa ATCC 9027 | 178000 | 0 | 15000/++ | 21000/++ | 90000/++ |
| | | 0,015 | 0/- | 0/- | 0/- |
| | | 0,03 | 0/- | 0/- | 0/- |
| | | 0,15 | 0/- | 0/- | 0/- |
| Candida albicans ATCC 10231 | 255000 | 0 | 17300/++ | 30000/++ | 2800/++ |
| | | 0,015 | 0/- | 0/- | 0/- |
| | | 0,03 | 0/- | 0/- | 0/- |
| | | 0,15 | 0/- | 0/- | 0/- |
| Aspergillus niger ATCC 16404 | 129000 | 0 | 22000/++ | 13000/++ | 8600/++ |
| | | 0,015 | 2970/++ | 0/- | 0/- |
| | | 0,03 | 0/- | 0/- | 0/- |
| | | 0,15 | 0/- | 0/- | 0/- |

EP 0 385 369 B1

## T a b e l l e   4

| Testkeime | Anfangsbelast. (KBE/ml) | Ergebnis der Reisolierung (Keimzahl/ml) nach: | | | | |
|---|---|---|---|---|---|---|
| | | 6 Std. | 24 Std. | 7 Tagen | 14 Tagen | 21 Tagen |
| E.coli ATCC 8739 | 200000 | 180000 | 300 | 0 | nicht bestimmt | |
| | | 170000 | 100 | 0 | nicht bestimmt | |
| Pseudomonas aerugi- nosa ATCC 9027 | 340000 | 322000 | 120 | 0 | nicht bestimmt | |
| | | 320000 | 100 | 0 | nicht bestimmt | |
| Staphylococcus aureus ATCC 6538 | 200000 | 280000 | 1500 | 0 | nicht bestimmt | |
| | | 170000 | 120 | 0 | nicht bestimmt | |
| Candida albicans ATCC 10231 | 170000 | 120000 | 50 | 0 | nicht bestimmt | |
| | | 90000 | 100 | 0 | nicht bestimmt | |
| Aspergillus niger ATCC 16404 | 600000 | 70000 | 500 | 10 | 12 | 0 |
| | | 90000 | 800 | 10 | 18 | 0 |

EP 0 385 369 B1

## T a b e l l e  5

| Testkeime | Anfangsbelast. (KBE/ml) | Ergebnis der Reisolierung (Keimzahl/ml) nach: | | | | |
|---|---|---|---|---|---|---|
| | | 6 Std. | 24 Std. | 7 Tagen | 14 Tagen | 21 Tagen |
| E.coli ATCC 8739 | 200000 | 280000 | 1400 | 0 | nicht bestimmt | |
| | | 220000 | 1200 | 0 | nicht bestimmt | |
| Pseudomonas aerugi-nosa ATCC 9027 | 700000 | 400000 | 410 | 0 | nicht bestimmt | |
| | | 380000 | 120 | 0 | nicht bestimmt | |
| Staphylococcus aureus ATCC 6538 | 190000 | 2000 | 80 | 0 | nicht bestimmt | |
| | | 2100 | 10 | 0 | nicht bestimmt | |
| Candida albicans ATCC 10231 | 220000 | 18000 | 130 | 0 | nicht bestimmt | |
| | | 20000 | 110 | 0 | nicht bestimmt | |
| Aspergillus niger ATCC 16404 | 130000 | 150000 | 18000 | 170 | 0 | n.b. |
| | | 140000 | 19000 | 130 | 10 | 0 |

EP 0 385 369 B1

**Tabelle 6**

| Testkeime | Anfangsbelast. (KBE/ml) | Ergebnis der Reisolierung (Keimzahl/ml) nach: | | | | |
|---|---|---|---|---|---|---|
| | | 6 Std. | 24 Std. | 7 Tagen | 14 Tagen | 21 Tagen |
| E.coli ATCC 8739 | 290000 | 22000 | 0 | nicht bestimmt | nicht bestimmt | |
| | | 2000 | 0 | nicht bestimmt | nicht bestimmt | |
| Pseudomonas aerugi-nosa ATCC 9027 | 300000 | 210000 | 0 | nicht bestimmt | nicht bestimmt | |
| | | 270000 | 12 | nicht bestimmt | nicht bestimmt | |
| Staphylococcus aureus ATCC 6538 | 180000 | 10000 | 0 | nicht bestimmt | nicht bestimmt | |
| | | 9000 | 0 | nicht bestimmt | nicht bestimmt | |
| Candida albicans ATCC 10231 | 110000 | 1100 | 0 | nicht bestimmt | nicht bestimmt | |
| | | 800 | 0 | nicht bestimmt | nicht bestimmt | |
| Aspergillus niger ATCC 16404 | 90000 | 100000 | 30000 | 0 | nicht bestimmt | nicht bestimmt |
| | | 80000 | 29000 | 0 | nicht bestimmt | nicht bestimmt |

## Patentansprüche

1. Verfahren zur antimikrobiellen Konservierung von flüssigen Tensiden und tensidhaltigen Lösungen oder Zubereitungen, dadurch gekennzeichnet, dass die flüssigen Tenside oder tensidhaltigen Lösungen oder Zubereitungen mit 0,01 bis 1,0 Gew.% N,N-Bis-(3-aminopropyl)-laurylamin versetzt werden.

**2.** Verfahren nach Patentanspruch 1, dadurch gekennzeichnet, dass die flüssigen Tenside oder tensidhaltigen Lösungen oder Zubereitungen mit 0,02 bis 0,5 Gew.% N,N-Bis-(3-aminopropyl)-laurylamin versetzt werden.

**3.** Verfahren nach Patentanspruch 1 oder 2, dadurch gekennzeichnet, dass das N,N-Bis-(3-aminopropyl)-laurylamin in Form einer 20-40%igen wässrigen Lösung eingesetzt wird.

**4.** Anwendung des Verfahrens nach mindestens einem der Ansprüche 1-3 zur antimikrobiellen Konservierung von anionischen flüssigen Tensiden oder deren Lösungen oder Zubereitungen.

**5.** Anwendung des Verfahrens nach mindestens einem der Ansprüche 1-3 zur antimikrobiellen Konservierung von tensidhaltigen Kühlschmierstoffen.

**6.** Anwendung des Verfahrens nach mindestens einem der Ansprüche 1-3 zur antimikrobiellen Konservierung von tensidhaltigen Kettengleitmitteln

**7.** Anwendung des Verfahrens nach mindestens einem der Ansprüche 1-3 zur antimikrobiellen Konservierung von tensidhaltigen Körperpflegemitteln.

**8.** Verwendung von N,N-Bis-(3-aminopropyl)-laurylamin zur Durchführung des Verfahrens gemäss einem der Ansprüche 1-3.

**Claims**

**1.** A process for antimicrobial preservation of liquid surfactants and surfactant-containing solutions or formulations, characterized in that to the liquid surfactants or surfactant-containing solutions or formulations 0.01 to 1.0% by weight N,N-bis-(3-aminopropyl)-laurylamine is added.

**2.** The process of claim 1, characterized in that to the liquid surfactants or surfactant-containing solutions or formulations 0.02 to 0.5% by weight N,N-bis-(3-aminopropyl)-laurylamine is added.

**3.** The process of claim 1 or 2, characterized in that N,N-bis-(3-aminopropyl)-laurylamine is used as 20-40% aqueous solution.

**4.** Use of the process of at least one of the claims 1-3 for antimicrobial preservation of anionic liquid surfactants or solutions or formulations thereof.

**5.** Use of the process of at least one of the claims 1-3 for antimicrobial preservation of surfactant-containing cooling lubricants.

**6.** Use of the process of at least one of the claims 1-3 for antimicrobial preservation of surfactant-containing chain lubricants.

**7.** Use of the process of at least one of the claims 1-3 for antimicrobial preservation of surfactant-containing body-care compositions.

**8.** Use of N,N-bis-(3-aminopropyl)-laurylamine for practice of the process of one of the claims 1-3.

**Revendications**

**1.** Procédé pour la conservation antimicrobienne d'agents tensioactifs liquides et de solutions ou de préparations contenant des agents tensioactifs, caractérisé en ce que les agents tensioactifs liquides ou les solutions ou préparations contenant des agents tensioactifs sont mis en réaction avec 0,01 jusqu'à 1,0 % en poids de N,N-bis-(3-aminopropyl)-laurylamine.

**2.** Procédé selon la revendication 1, caractérisé en ce que les agents tensioactifs liquides ou les préparations ou solutions contenant des agents tensioactifs liquides sont mis en réaction avec 0,02 jusqu'à 0,5 % en poids de N,N-bis-(3-aminopropyl)-laurylamine.

3. Procédé selon la revendication 1 ou 2, caractérisé en ce que la N,N-bis-(3-aminopropyl)-laurylamine est mise en oeuvre sous forme d'une solution aqueuse à 20-40 %.

4. Application du procédé selon au moins l'une des revendications 1-3 pour la conservation antimicrobienne d'agents tensioactifs liquides anioniques ou leurs préparations ou solutions.

5. Utilisation du procédé selon au moins l'une des revendications 1-3 pour la conservation antimicrobienne de réfrigérants lubrifiants contenant des agents tensioactifs.

6. Application du procédé selon au moins l'une des revendications 1-3 pour la conservation antimicrobienne de lubrifiants pour chaînes contenant des agents tensioactifs.

7. Application du procédé selon au moins l'une des revendications 1-3 pour la conservation antimicrobienne de produits de soins corporels contenant des agents tensioactifs.

8. Utilisation de la N,N-bis-(3-aminopropyl)-laurylamine pour la réalisation du procédé selon l'une des revendications 1-3.